# EUROPEAN PATENT APPLICATION

(11) **EP 4 285 803 A1**
(43) Date of publication of application: **06.12.2023**
(21) Application number: 21916955.4
(22) Date of filing: 23.03.2021
(51) Int. Cl.: A47L 11/30

(54) **DEHUMIDIFICATION TRAY FOR USE IN CLEANING DEVICE, AND CLEANING DEVICE**

(30) Priority: 05.01.2021 CN 202120015588 U
(71) Applicant: Qiu, Rixing, Changzhou, Jiangsu 213251 (CN)
(72) Inventor: QIU, Riju, Changzhou, Jiangsu 213251 (CN); QIU, Rixing, Changzhou, Jiangsu 213251 (CN)
(74) Representative: Chung, Hoi Kan
(86) International application number: PCT/CN2021/082431
(87) International publication number: WO 2022/147904

(57) **Abstract**

The present disclosure discloses a dehumidifying tray for a cleaning apparatus, which is provided with a tray body and a dehumidifying device, wherein the dehumidifying device drives air into a rolling brush chamber to dry the rolling brush when the cleaning apparatus is placed on the tray body. The dehumidifying device comprises a dehumidifying duct and a dehumidifying fan, and when the cleaning apparatus is placed on the tray body, the dehumidifying duct is in fluid communication with the rolling brush chamber, and the dehumidifying fan is capable of driving the air into the rolling brush chamber through the dehumidifying duet, thereby dehumidifying the rolling brush. This disclosure also provides a cleaning apparatus.

## Description

### TECHNICAL FILED

The present disclosure relates to cleaning apparatus technology, specifically, to a dehumidifying tray for cleaning apparatus and a cleaning apparatus.

### BACKGROUND

Wet cleaning apparatus is a common household cleaning apparatus that cleans the floor by spraying water onto the floor or a rolling brush and then cleaning the floor by the rolling brush and then sucking the sewage into the cleaning apparatus. A wet cleaning apparatus comprises a floor brush mechanism (e.g., a floor brush head), a clean water tank, a sewage tank and a sewage suction fan. The sewage suction fan drives the sewage to flow into the sewage tank. After the cleaning apparatus completes the cleaning work, it is usually placed in a static way to make the rolling brush of the floor brush mechanism dry naturally. If the rolling brush fails to get dry quickly, it may be stink and cause odor in the home environment. How to make the rolling brush dry quickly and avoid the brush from getting sticky during rest is one of the technical optimization essentials of the cleaning apparatus. In view of this, this present disclosure is hereby proposed.

### SUMMARY

The technical problem to be solved by the present disclosure is how to quickly dry the rolling brush of the cleaning apparatus, so as to prevent the rolling brush from getting sticky during rest.

In order to achieve the above purpose, a dehumidifying tray for cleaning apparatus is provided. The cleaning apparatus comprises a cleaning head, a suction device and a sewage suction channel. The cleaning head comprises a rolling brush chamber and a brush that is built into the brush chamber. The brush chamber is located at an upstream position of the sewage suction channel. The suction device is located at a downstream position of the sewage suction channel and is configured to generate negative pressure in the sewage suction channel. The dehumidifying tray comprises: a tray body and a dehumidifying device. The tray body has a receiving groove formed by a recessed portion. The receiving groove is adapted to the cleaning head for placing a cleaning apparatus in a non-working state. The receiving groove is adapted for placing a cleaning apparatus in a non-working state. The dehumidifying device is disposed on the tray body and is capable of introducing air into the brush chamber to dry the rolling brush when the cleaning apparatus is placed on the tray body. The dehumidifying device comprises a dehumidifying duct and a dehumidifying fan. The dehumidifying duct is in fluid communication with the brush chamber when the cleaning apparatus is placed on the tray body. The air is driven by the dehumidifying fan from the dehumidifying duct into the brush chamber.

The disclosure further provides a cleaning apparatus. The cleaning apparatus comprises a cleaning head, a suction device and a sewage suction channel. The cleaning head comprises a brush chamber and a brush built into the brush chamber. The brush chamber being located at an upstream position of the sewage suction channel, the suction device being located at a downstream position of the sewage suction channel and configured to generate negative pressure in the sewage suction channel. The cleaning apparatus is adapted for being placed on the dehumidifying tray as discussed. In addition to the first air section that supplies air to flow to the moisture discharge air outlet, the air outlet section of the moisture discharge duct is also provided with a second air section that guides the air that deviates from the first air section to flow back to the first air section. The second air section is an arc-shaped structure. The arc-shaped second air section enables the air to flow back in the second air section, which helps to reduce the noise generated by impact on the duct wall, thereby reducing the overall noise when the fan is working.

Preferably, a filter is provided in the dirty liquid suction channel upstream of the suction device. When the cleaning device is placed on the tray body, the dehumidification air channel and the filter are in fluid communication, and the air flows from the roller brush chamber through the dirty liquid suction channel into the filter to dry the filter.

Preferably, the cleaning head also has a humidity sensor.

Preferably, the roller brush is configured such that when the cleaning device is placed on the dehumidification tray, it can be controlled to rotate so that its different areas are opposite to the air outlet of the dehumidification duct.

As a result of the above technical solutions, the present disclosure has the following beneficial effects: when the dehumidifying tray of the present disclosure is supporting the cleaning apparatus, the dehumidifying fan can drive the air to flow into the sewage suction channel, so as to dehumidify the rolling brush, which benefits the quick dry of the rolling brush and prevents it from sticky during rest. The air flowing into the sewage suction channel can also dehumidify the filter located at an outlet end of the sewage suction channel. When the dehumidifying fan drives the air to flow out through the outlet of the dehumidifying duct, it also simultaneously drives the water vapor in the water collection tank to disperse.

In order to solve the problem of the large working noise existing in the prior art sewage suction fan, the disclosure can also have the following improvements.

More specifically, the suction device may comprise a fan housing. The fan housing having a moisture discharge duct and a cooling duct. The air outlet section of the moisture discharge duct comprises a first air section for direct flow of air to the outlet of the moisture discharge duct, and a second air section for guiding the wind deviating from the first air section to return back to the first air section. The second air section is arc-shaped.

Preferably, the fan housing comprises an inner housing assembly and an outer housing assembly. The outer housing assembly comprises an inner cavity for accommodating the inner housing assembly. The inlet section of the moisture discharge duct is located at the inner housing assembly, and the outlet section of the moisture discharge duct is located at the housing assembly. A transition air section is located between the inner housing assembly and outer housing assembly and connects the inlet section and outlet section of the moisture discharge duct.

Preferably, the fan housing comprises an inner housing assembly and an outer housing assembly, the outer housing assembly having an inner cavity for accommodating the inner housing assembly. The inlet section of the cooling duct is located at the outer housing assembly and the outlet section of the cooling duct is located at the inner housing assembly.

Preferably, the air outlet section of the cooling duct is connected to the air inlet section of the moisture discharge duct.

Preferably, the inner housing assembly comprises a first inner housing and a second inner housing. The first inner housing has a first cavity for accommodating a dehumidifying fan and a second cavity for accommodating a cooling fan. The first cavity is on the lower side of the second cavity. The second inner housing is attached to the lower side of the first inner housing, and the end of the air outlet section of the cooling duct is located between the outer wall of the first inner housing and the inner wall of the second inner housing.

Preferably, the housing assembly comprises a first outer housing, a second housing and a housing cover. The first outer housing is mounted on the upper side of the second outer housing and a cavity is formed therebetween to accommodate the inner housing assembly. The housing cover is mounted on the upper side of the first housing and an air outlet section of the moisture discharge duct is formed therebetween.

By adopting the above technical solutions, the air outlet section of the moisture discharge duct of the suction device comprises not only the first air section from which the air flows to the moisture discharge outlet, but also a second air section that guides the air to deviate from the first air section to return back to the first air section, and the second air section is arc-shaped. The arc-shaped second air section enables the air to flow back in the second air section, which helps to reduce the noise generated by the impact on the wall of the duct, thereby reducing the overall noise of the fan during its operation.

### BRIEF DESCRIPTION OF THE DRAWINGS

In order to illustrate the technical solutions of the embodiments of the present disclosure more clearly, the drawings of the embodiments will be briefly described as below. Obviously, the drawings in the following description only relate to some embodiments of the present disclosure, and are not to limit the present disclosure.
FIG. 1 illustrates a schematic diagram of a cleaning apparatus according to one embodiment;
FIG. 2 and FIG.3 illustrates an exploded view of the cleaning apparatus according to one embodiment;
FIG. 3 illustrates an exploded view of the cleaning apparatus according to one embodiment of the present disclosure from a different perspective;
FIG. 4 illustrates a cross-sectional view of a cleaning apparatus according to one embodiment of t;
FIG. 5 illustrates a cross-sectional view of the cleaning apparatus according to one embodiment;
FIG. 6 illustrates an exploded view corresponding to FIG. 5.
FIG. 7 and FIG. 8, separately, illustrates an exploded view of the cleaning apparatus from a different perspective;
FIG. 9 and FIG. 10 illustrate a schematic diagram and an exploded diagram of a clean water tank, respectively;
FIG. 11 illustrate an exploded view of the clean water tank;
FIG. 12 and FIG. 13 illustrate a schematic diagram and an exploded view of a sewage tank, respectively;
FIG. 14 illustrates a sectional view of the sewage tank;
FIG. 15 illustrates a schematic diagram of the cleaning head in different views;
FIG. 16 illustrates a first exploded view of the cleaning head;
FIG. 17 illustrates a second exploded view of the cleaning head;
FIG. 18 illustrates a cross-sectional view of the cleaning head;
FIG. 19 illustrates a schematic view of the brush roll cover;
FIG. 20 and FIG. 21 illustrates a schematic diagram and an exploded view of the dehumidifying tray, respectively;

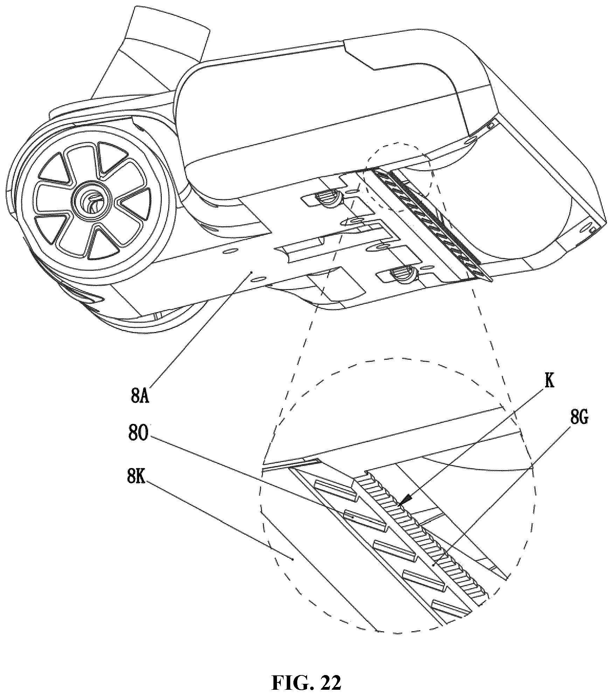

FIG. 22 illustrates a schematic diagram of a cleaning head according to another embodiment;
FIG. 23 illustrates a diagram of the attachment process of a scraper and the connecting member;
FIG. 24 illustrates a schematic diagram of a suction device;
FIG. 25 illustrates a first exploded view of the suction device;
FIG. 26 illustrates a sectional view of the suction device;
FIG. 27 illustrates a second exploded view corresponding to FIG. 26;
FIG. 28 illustrates a schematic diagram of a first housing;
FIG. 29 illustrates a cross-sectional view of a cleaning head according to another embodiment;
FIG. 30 illustrates an exploded view of a dehumidifying tray according to another embodiment;
FIG. 31 illustrates an exploded view of a dehumidifying tray according to another embodiment.

The accompanying drawings are marked as below:
cleaning apparatus 1, dehumidifying tray 2, body 3, clean water tank 4, sewage tank 5, suction device 6, filter 7, cleaning head 8, battery mechanism 9, handle assembly 10.

### DETAILED DESCRIPTION OF THE EMBODIMENTS

In order to make the purpose, technical solutions and advantages of the embodiments of the present disclosure clearer, the present disclosure will be described in further detail below in conjunction with the accompanying drawings. The components according to the embodiments of the present disclosure generally described and illustrated in the accompanying drawings herein can be arranged and designed in various different configurations. Based on the embodiments of the present disclosure, all other embodiments obtained by a person of ordinary skill in the art without making creative labor shall fall within the scope of protection of the present disclosure.

Hereinafter, some embodiments of the present disclosure are described in detail with reference to the accompanying drawings. The features in the embodiments described below can be combined with each other in a non-conflicting manner.

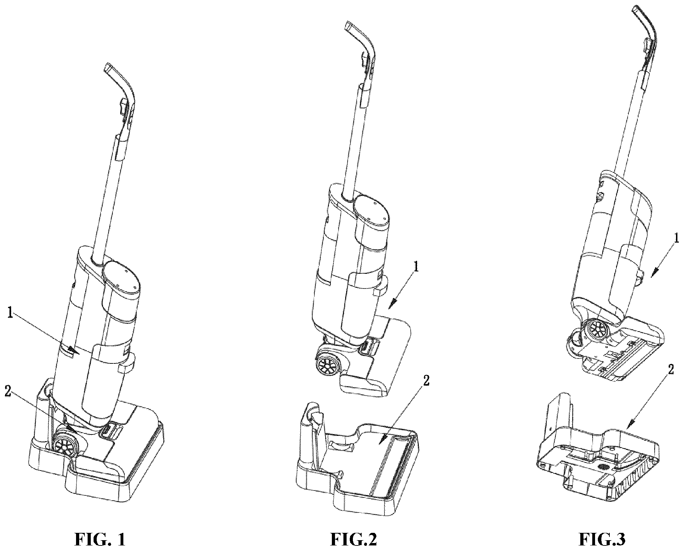

Combined with FIG. 1 to FIG. 4, in one embodiment, the cleaning apparatus of the present disclosure comprises a cleaning apparatus 1 and a dehumidifying tray 2. The cleaning apparatus 1 can be separated from the dehumidifying tray 2 as a whole. When the cleaning apparatus 1 is supported on the dehumidifying tray 2, the dehumidifying tray 2 can dehumidify the cleaning apparatus 1 on the one hand, and can also charge the cleaning apparatus 1 on the other hand.

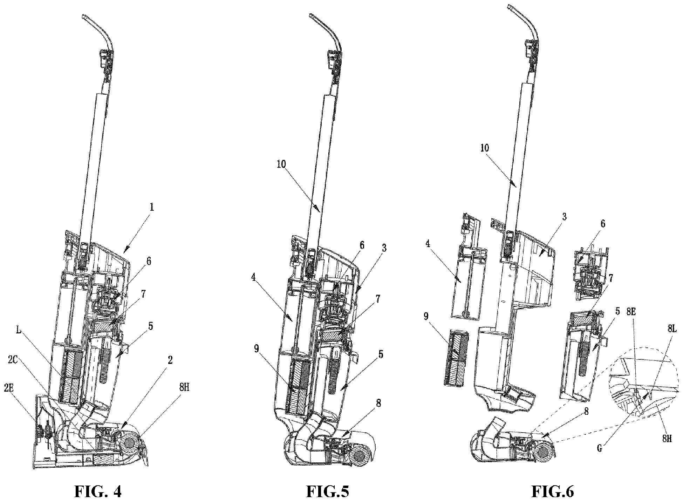

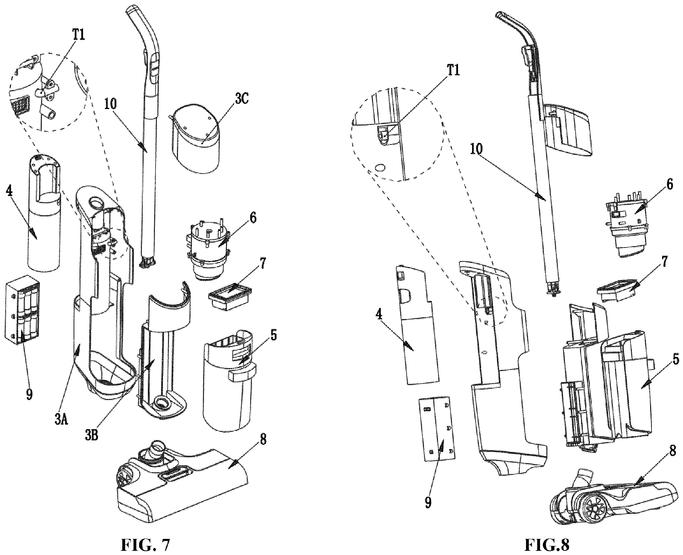

**[Cleaning apparatus]** With reference to FIG. 5 to FIG. 8, the cleaning apparatus 1 comprises a body 3, a clean water tank 4, a sewage tank 5, a suction device 6, a filter 7, a cleaning head 8, a battery mechanism 9, and a handle assembly 10.

**[Body]** The body 3 comprises a first housing 3A, a second housing 3B and a third housing 3B. The clean water tank 4 is mounted on the first housing 3A. The sewage tank 5 and the filter 7 are mounted on the second housing 3B. The suction device 6 is mounted between the second housing 3B and the third housing 3B. The cleaning head 8 is mounted on the lower side of the first housing 3A. The battery mechanism 9 is mounted on the first housing 3A.The handle assembly 10 being mounted on the first housing 3A. A water conduit T1 is mounted on the first housing 3A.

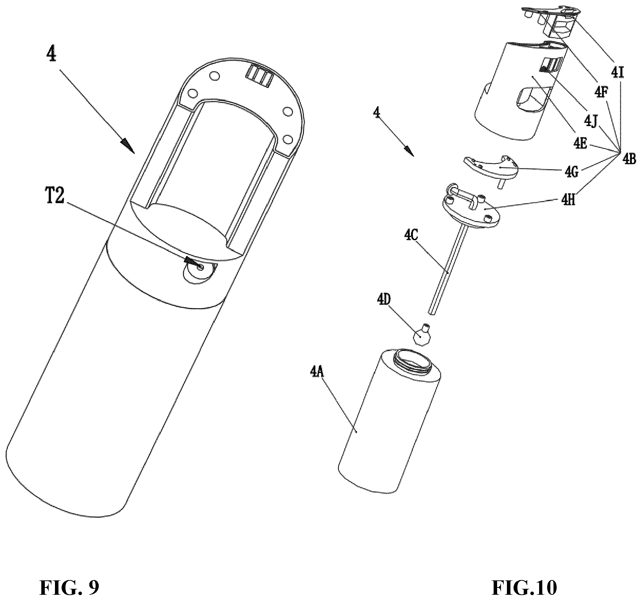

**[Clean water tank]** With reference to FIG. 9 to FIG. 11, the clean water tank 4 has a water outlet end T2 that fits with the water conduit T1, and the clean water tank 4 can be detachably mounted on the first housing 3A as a whole. Referring to FIG. 9 to FIG. 11, the clean water tank 4 includes a first water storage component 4A, a first cover component 4B, a water conduit hose 4C and a counterweight 4D. The first water storage component 4A and the first cover component 4B cooperate to form the main body of the clean water tank. The first water storage component 4A and the first cover component 4B cooperate through a threaded structure, and the first cover component 4B can be detachably from the first water storage component 4A as a whole. In this embodiment, the water conduit hose 4C has a water inlet end extending to the lower side of the inner cavity of the clean water tank body and a water outlet end connected to the external space. In this embodiment, the water conduit hose 4C is installed on the first cover assembly 4B, and the counterweight 4D is installed on the water inlet end of the water conduit hose 4C. The outer circumference of the upper water inlet neck of the water purification assembly 4A has external threads. The first cover assembly 4B includes a main cover 4E, a top cover 4F, a handle 4G, a bottom cover 4H, a button 4I, a spring member 4J, and a first sealing gasket. 4K and the second sealing gasket 4L, the main cover 4E has a grasping cavity for hand gripping, the top cover 4F is installed on the upper side of the main cover 4E, the bottom cover 4H is installed on the lower side of the main cover 4E, and the handle 4G is installed in the grasping cavity of the main cover 4E for the user to hold. The button 4I is installed on the main cover 4E and the top extends upward from the main cover 4E. The spring member 4J is in contact between the button 4I and the main cover 4E. The lower side of the bottom cover 4H has a socket portion that matches the upper water inlet neck of the first water storage component 4A, the inner circumference of the socket portion has internal threads, and the first sealing gasket 4K is located on the lower side of the bottom cover 4H And is located on the inner peripheral side of the socket portion, and the second sealing gasket 4L is located on the lower side of the bottom cover 4H and is sleeved on the outer peripheral side of the socket portion. The water conduit hose 4C passes through the bottom cover 4H. The water outlet end T2 of the water conduit hose 4C is located outside the main cover 4E. The water inlet end of the water conduit hose 4C extends into the inner cavity of the first water storage component 4A. The counterweight 4D is used to prevent the water-conducting hose 4C from curling, and the counterweight 4D is provided with a water channel connected to the water-conducting hose 4C, thereby giving way to the water inlet of the water inlet end of the water-conducting hose 4C. In this embodiment, the upper side of the counterweight 4D is sleeved in the water conduit hose 4C, and the lower side has a spherical structure.

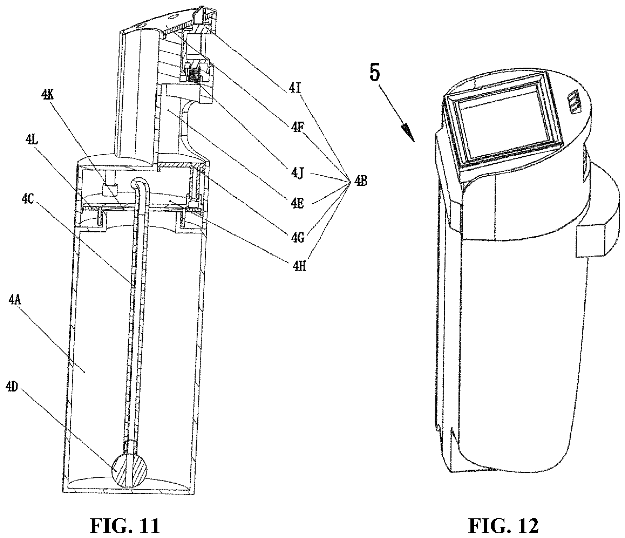

**[Sewage tank]** With reference to FIG. 12 to FIG. 14, the sewage tank 5 comprises a water storage assembly 5A, a cover assembly 5B and a buoy member 5C. The inner cavity of the second water storage assembly 5A has a sewage entry channel for sewage to enter. The cover assembly 5B is detachably mounted on the water storage assembly 5A. The water storage assembly 5A has a filter holder for mounting the filter 7, and a buoy holder for mounting the buoy member 5C. The buoy member 5C is mounted on the buoy holder and is capable of slide up and down along the buoy holder. The buoy member 5C rising as the sewage level in the water storage assembly 5A rises.

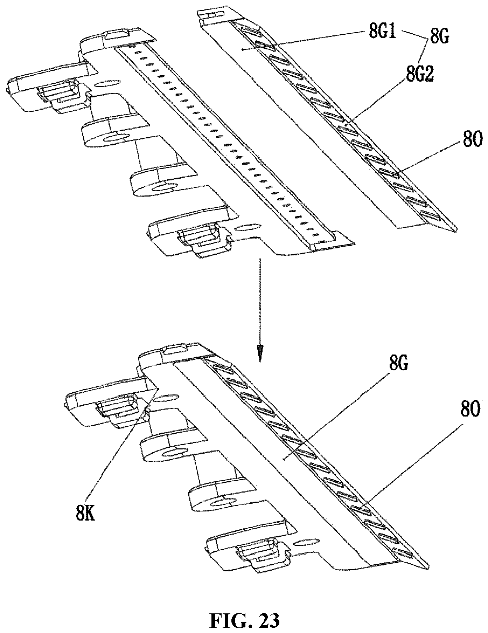

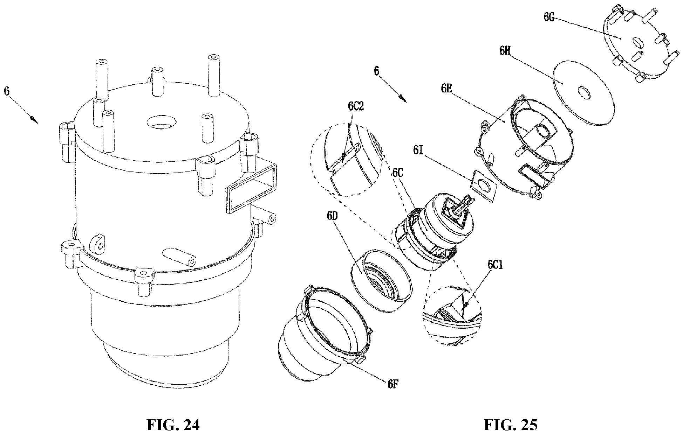

**[Suction device]** The suction device 6 is located downstream of the sewage suction channel L and is configured to generate negative pressure in the suction channel. Specifically, the suction device 6 is mounted between the second housing 3B and the third housing 3B.The suction device 6 is connected to the filter 7. The suction device 6 can be disassembled by removing the third housing 3B. With reference to FIG. 24 to FIG. 28, the suction device 6 is a suction fan, which comprises a suction fan housing. The suction fan housing has a moisture discharge duct Q1 and a cooling duct Q2. Specifically, the suction fan housing comprises an inner housing assembly 6A and an outer housing assembly 6B, with the outer housing assembly 6B having an inner cavity for accommodating the inner housing assembly 6A. The inlet section Q11 of the moisture discharge duct Q1 is located in the inner housing assembly 6A, the outlet section Q12 of the moisture discharge duct Q1 is located in the housing assembly 6B, the transition air section Q13 is located between the inner housing assembly 6A and the housing assembly 6B and communicates with the inlet section Q11 and the outlet section Q12 of the moisture discharge duct Q1 (as shown in FIG. 25, the first inner housing 6C has an aperture 6C1 for moisture to pass through), the outlet section Q12 comprises the first air section Q121 that provides the air to direct flows to the moisture discharge air outlet 6E2, and the second air section Q122 that guides the air deviating from the first air section Q121 to flow back to the first air section Q121, the second air section Q122 is arc-shaped, and the arc-shaped second air section enables the air to flow back in the second air section, which helps reduce the noise generated by the impact of the duct wall, thereby reducing the overall noise when the dehumidifier fan during operation. The inlet section Q21 of the cooling duct Q2 is located in the outer housing assembly 6B, and the outlet section Q22 of the cooling duct Q2 is located in the inner housing assembly 6A, and the outlet section Q22 of the cooling duct Q2 is connected to the inlet section Q11 of the moisture discharge duct Q1. The inner housing assembly 6A comprises a first inner housing 6C and a second inner housing 6D, and the first inner housing 6C has a first cavity P1 in which a dehumidifying fan (not shown) is mounted and a second cavity P2 in which a cooling fan is installed (not shown), the first cavity P1 is located on the lower side of the second cavity P2, the second inner housing 6D is snapped to the lower side of the first inner housing 6C, and the end of the outlet section Q22 of the cooling duct Q2 is located between the outer wall of the first inner housing 6C and the inner wall of the second inner housing 6D (as shown in FIG. 23, the outer wall of the first inner housing 6C has a recessed section 6C2). The housing assembly 6B comprises a first outer housing 6E, a second outer housing 6F and an outer housing cover 6G. The first housing 6E has a moisture inlet 6E1, a moisture discharge vent 6E2 and an arc-shaped air guiding surface, which is opposite to the second air section Q122. The first outer housing 6E is mounted on the upper side of the second outer housing 6F and a cavity is formed therebetween to accommodate the inner housing assembly 6A, the outer housing cover 6G is mounted on the upper side of the first outer housing 6E and the outlet section Q12 of the moisture discharge duct Q1 is formed therebetween. A gasket 6H is provided between the outer housing cover 6G and the first outer housing 6E, and a gasket 6I is provided between the first inner housing 6C and the first outer housing 6E.

**[Filter]** The filter 7 is located at the upstream position of the suction device 6 along the sewage suction channel L. The filter 7 is mounted on the filter holder of the water storage assembly 5A and the outlet of the sewage suction channel L. The filter 7 is located at the front side of the inlet of the suction device 6 to prevent sewage from entering the suction device 6. The filter 7 may be a HEPA assembly. When the cleaning apparatus 1 is placed on the tray body 2, the dehumidifying duct N1 and the filter 7 are in fluid communication, and air flows from the rolling brush chamber through the sewage suction channel to the filter 7 to dry the filter 7, as further described later in connection with the specific structure of the tray body 2.

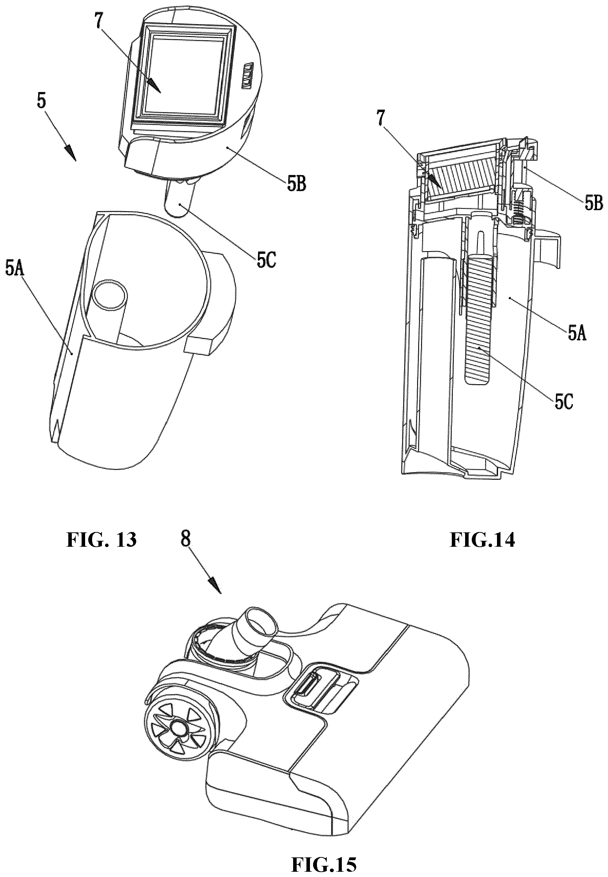

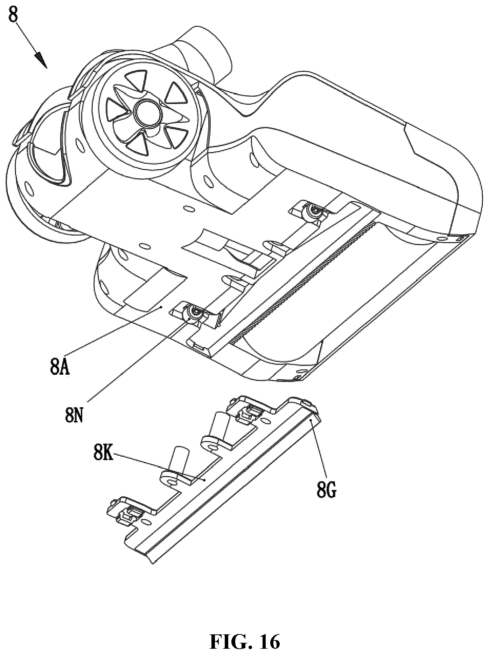

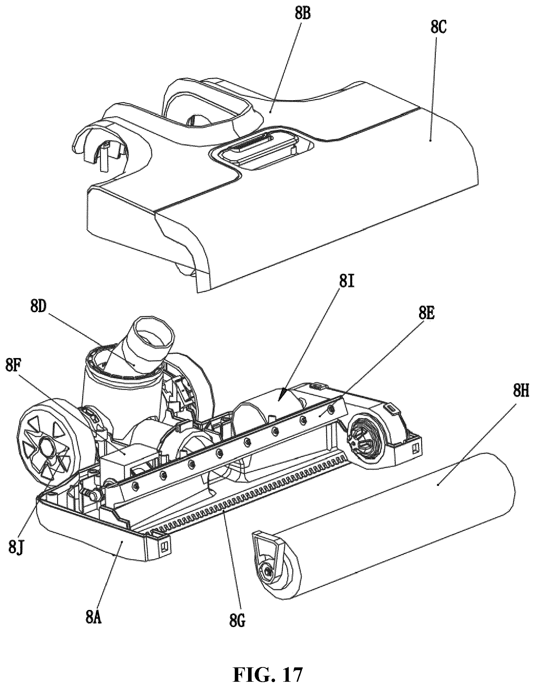

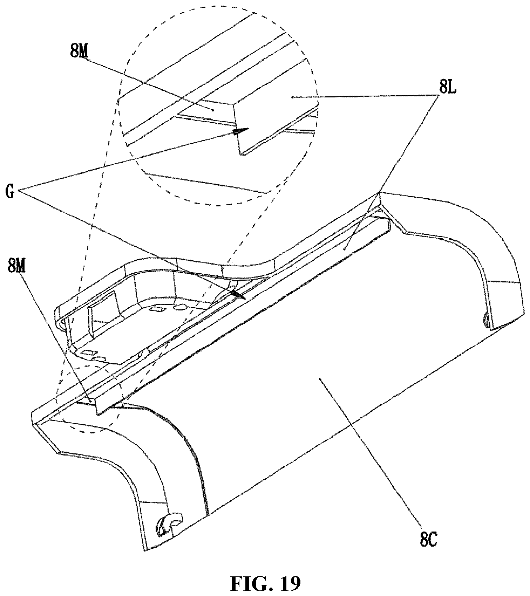

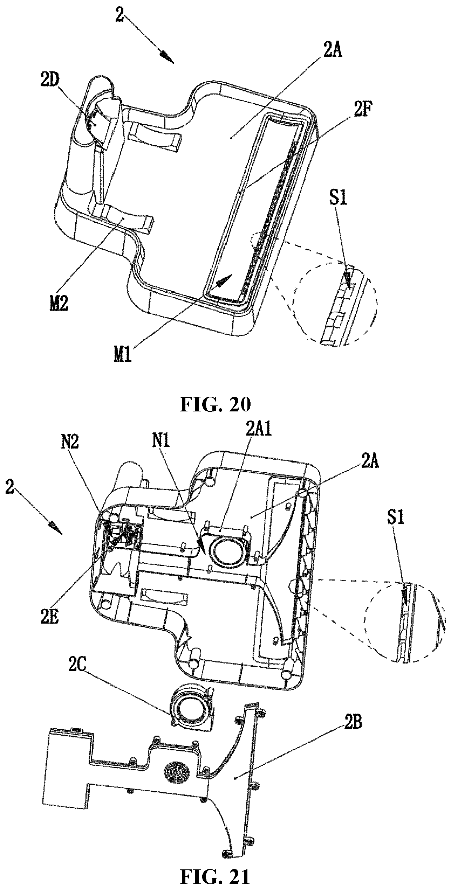

**[Cleaning head]** With reference to FIG. 15 to FIG. 19, the cleaning head 8 includes a floor brush bottom cover 8A, a floor brush top cover 8B, a rolling brush cover 8C, a sewage conduit 8D, a spraying member 8E, a water pump 8F, a scraper 8G, a rolling brush 8H, a drive assembly 8I, rollers 8J, a connecting member 8K, a flow guide 8L, a flow blocking member 8M, and auxiliary wheels 8N. The floor brush bottom cover 8A, the floor brush top cover 8B, and the rolling brush cover 8C fit together to form the floor brush housing. The floor brush top cover 8B is mounted on the floor brush bottom cover 8A, and the rolling brush cover 8C is detachably mounted on the floor brush top cover 8B. The sewage conduit 8D is connected to the sewage inlet channel of the sewage tank 5, the water inlet of the spraying member 8E is connected to the water outlet of the water pump 8F, and the water inlet of the water pump 8F is connected to the water conduit T1 through a pipeline (not shown), and the floor brush housing has a rolling brush chamber for the brush 8H, and the rolling brush chamber is located upstream of the sewage suction channel L, and the rolling brush 8H is mounted on the floor brush bottom cover 8A, and the drive assembly 8I drives the rolling brush 8H to roll, and a pair of rollers 8J are mounted on the rear side of the floor brush bottom cover 8A. The scraper 8G is usually made of soft rubber material, and the scraper 8G has a fixed portion 8G1 which is secured to the floor brush bottom cover 8A, as well as a liquid scraping portion 8G2 which is capable of contacting the ground, the fixed portion 8G1 is secured to the lower side of the floor brush bottom cover 8A through the connecting member 8K, auxiliary wheels 8N are installed between the connecting member 8K and the floor brush bottom cover 8A, a gap is provided between the liquid scraping portion 8G2 and the rolling brush 8H for the sewage to enter the sewage suction channel L, a sewage over-hole K is provided on the close part of the floor brush bottom cover 8A that is near the upper side of the liquid scraping portion 8G2 where the sewage over-hole K is to provide for the sewage to move upward into the sewage suction channel L. In this embodiment, the close part of the floor brush bottom cover 8A that is near the upper side of the liquid scraping portion 8G2 is comb-shaped, and the sewage over-hole K can prevent the sewage from accumulating at the angle between the scraper and the floor brush bottom cover. The flow guide 8L is set on the lower side of the rolling brush cover 8C and is suspended on the upper side of the rolling brush 8H. The flow guide 8L has a flow guide surface G on which the spray water is spread and guided to the brush 8H, and the spray port of the spraying member 8E faces the flow guide surface G. In this embodiment, the spray port of the spraying member 8E is tilted upward, so that the spray water is sprayed to the upper side of the flow guide surface G obliquely. The flow blocking member 8M is also set on the lower side of the rolling brush cover 8C, and the flow blocking member 8M is set on both sides of the flow guide surface G of the flow guide member 8L, so as to limit the expansion range of the spray water in the length direction of the rolling brush 8H. Combined with FIGS. 20 to 21, in the cleaning head according to another embodiment, the rear side of the scraping section 8G2 is provided with a plurality of support portions 8O that cause the bottom end of the scraping section 8G2 to overhang when the scraping section 8G2 is moved backward, and the plurality of support portions 8O are provided at intervals along the length direction of the scraper 8G, and there are sewage-passing gap between adjacent support portions 8O, the support portions 8O are strip-shaped, and when the cleaning head is moved backward, the support portions 8O cause the bottom end of the scraping section overhang, and the contact surface between the bottom end of the scraping section 8G2 and the ground is small, and the sewage-passing gaps are provided between adjacent support portions 8O so as to prevent sewage from being driven by the scraper, thus helping to reduce the generation of stains.

**[Battery mechanism]** The battery mechanism 9 is configured to supply power to the cleaning apparatus. The battery mechanism 9 may be a rechargeable lithium battery.

**[Handle assembly]** The handle assembly 10 is mounted on the first housing 3A. The handle assembly 10 comprises a control button to control the operation of the cleaning apparatus 1.

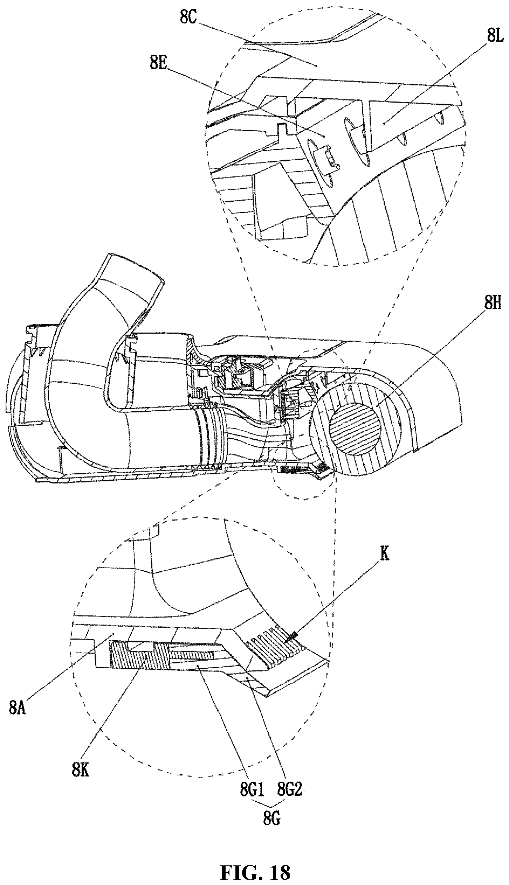

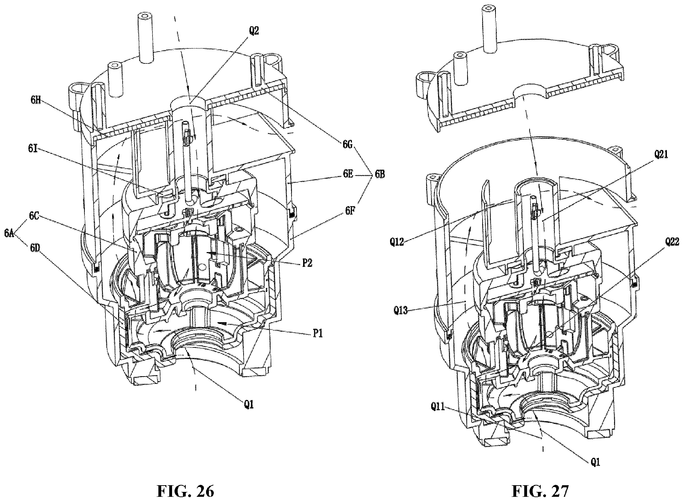

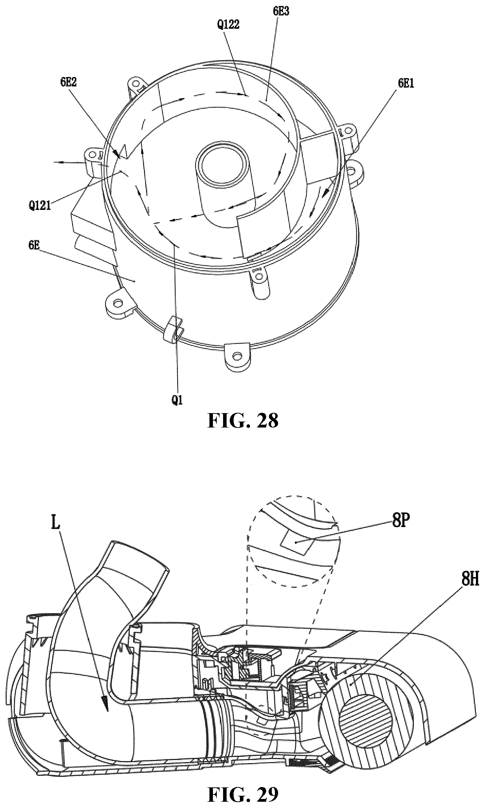

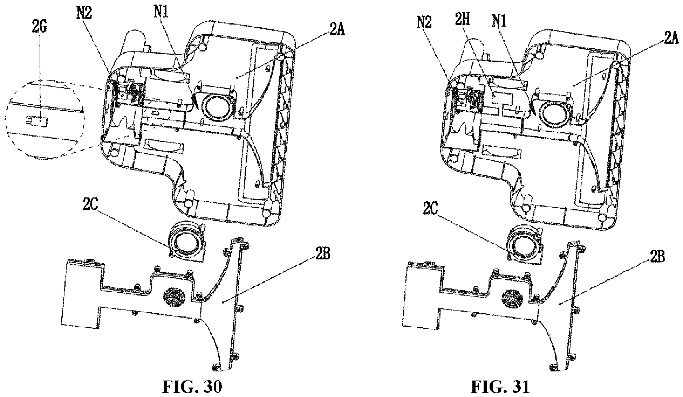

**[Dehumidifying tray]** With reference to FIG. 20 and FIG. 21, the dehumidifying tray 2 comprises a first tray housing 2A, a second tray housing 2B, a dehumidifying fan 2C, a charging valve 2D, a charging module 2E, and a seal 2F. The first tray housing 2A and the second tray housing 2B fit together to form the tray body, and the first tray housing 2A and the second tray housing 2B have a dehumidifying duct N1 and a mounting cavity N2, and they are in communication with each other. With reference to FIG. 20, the first tray housing 2A is the main housing of the dehumidifying tray, where the upper side of its bottom wall has a receiving groove formed by the recessed portion, and the lower side of its bottom wall is integrally formed with a duct baffle 2A1 for defining the area of the dehumidifying duct N1, the dehumidifying fan 2C is located in the area that is enclosed by the duct baffle 2A1, the receiving groove is adapted to the cleaning head to be adapted for placing the cleaning apparatus 1 in a non-working state, a plurality of through-holes are formed in the bottom wall of the first tray housing 2A, the plurality of through-holes being adapted for directing air flow to the rolling brush 8H, the plurality of through-holes serving as the outlet S1 of the dehumidifying duct N1, the receiving groove being connected to the dehumidifying duct via the through-holes, the upper side of the first tray housing 2A having a water collection tank M1 for holding the sewage remaining in the rolling brush 8H when supporting the cleaning apparatus 1 (i.e., the position of the water collection tank M1 is adapted to the position of the rolling brush 8H), and the roller support portion M2, the water collection tank M1 is formed by a further recess from the bottom of the receiving groove. The second tray housing 2B is a duct member that fits with the first tray housing 2A (main housing) to form a dehumidifying duct N1, the second tray housing 2B is secured to the lower side of the bottom wall of the first tray housing 2A and the pre-set gap between the bottom wall is formed as a dehumidifying duct N1, specifically, the lower side of the first tray housing 2A has a plurality of studs, and the second tray housing 2B has matching screw holes. The second tray housing 2B is connected to the first tray housing 2A by screws, and thus being secured to the duct baffle 2A1. The dehumidifying duct N1 has a gradually enlarged front portion to allow air to flow evenly to the rolling brush 8H within the length range of the rolling brush 2C, with the front portion extending forward away from the dehumidifying fan 2C. The air outlet S1 of the dehumidifying duct N1 is located at the front side of the water collection tank M1 (as in FIG. 18, in this embodiment, the air outlet S1 is configured at the wall of the water collection tank M1, and the air outlet S1 can also be adjusted to be configured at the transition between the wall of the water collection tank M1 and the bottom of the receiving groove), and there are a plurality of air outlets S1, and the plurality of air outlets S1 are arranged at intervals along the direction corresponding to the length direction of the rolling brush 8H. A sealing member 2F is provided on the peripheral side of the water collection tank M1, and when the cleaning apparatus 1 is placed on the dehumidifying tray, the sealing member 2F is configured between the water collection tank M1 and the rolling brush chamber as a seal to prevent the air that is configured to dry the rolling brush from leaking. The dehumidifying fan 2C is mounted in the dehumidifying duct N1 (the two cooperate to form a dehumidifying device so as to drive air into the rolling brush chamber when the cleaning apparatus is placed on the tray body) and the charging module 2E is mounted in the mounting cavity N2. The outlet of the dehumidifying fan 2C is fluidly communicated with the dehumidifying duct (N1) so that the dehumidifying fan 2C drives air through the rolling brush chamber into the sewage suction channel L to dehumidify the rolling brush 8H and the filter 7 (when the cleaning apparatus is placed on the tray body, the dehumidifying duct N1 is fluidly communicated with the rolling brush chamber), the dehumidifying fan 2C drives the air to flow through the air outlet S1 of the dehumidifying duct N1 and in the meanwhile disperse moisture from the water collection tank M1 and cool down the charging module 2E. With reference to FIG. 27, in other embodiments, in order to work with the dehumidifying fan 2C, the cleaning head 8 also has a humidity sensor 8P, where the humidity sensor 8P is configured in the sewage suction channel L which is located at the downstream position of the rolling brush 8H. The dehumidifying tray can control the operating state of the dehumidifying fan based on the detected information by the humidity sensor 8P, and only when the cleaning apparatus is placed on the tray body of the dehumidifying tray, the humidity sensor 8P detects the humidity of the air flowing through the rolling brush 8H for generating a control signal to control the start/stop of the dehumidifying fan 2C. When the humidity sensor 8P detects that the air humidity is less than the preset value, the dehumidifying fan 2C is controlled to turn off, and when the humidity sensor 8P detects that the air humidity is greater than the preset value, the dehumidifying fan 2C can be controlled to self-start. When the cleaning apparatus 1 is placed on the dehumidifying tray 1, the rolling brush 8H can be controlled to rotate so that its different areas are opposite to the air outlet S1 of the dehumidifying duct N1, and the speed of the rolling brush 8H at this time can be much lower than the speed of normal operation, for example, it can rotate one revolution every minute. With reference to FIG. 28, in another embodiment, the dehumidifying duct N1 of the dehumidifying tray 2 is configured with a heating device 2G, which heats the air to improve the drying efficiency of the brush, the heating device can be for example PTC heating, and it can be provided with another temperature control device (such as a fuse or a temperature detector) to be used in conj unction with the heating device 2G, in order to control the heating temperature of the heating device 2G. With reference to FIG. 31, in yet another embodiment, the tray body of the cleaning head 8 is configured with a negative ion generator 2H for sterilization and deodorization, and the negative ion output port of the negative ion generator 2H is connected to the dehumidifying duct N1.

The above mentioned is only specific embodiments of the present disclosure, but the scope of protection of the present disclosure is not limited to it. Any changes or substitutions that can be easily thought of by a person skilled in the art within the technical field disclosed by the present disclosure shall be covered by the scope of protection of the present disclosure. Therefore, the scope of protection of the present disclosure shall be subject to the scope of protection of the claims.

## Claims

1. A dehumidifying tray for a cleaning apparatus, wherein the cleaning apparatus comprises a cleaning head (8), a suction device (6) and a sewage suction channel (L), wherein the cleaning head has a rolling brush chamber and a rolling brush (8H) built into the brush chamber; wherein the rolling brush chamber is located in an upstream position of the sewage suction channel (L); the suction device (6) is located in a downstream position of the sewage suction channel (L) and is configured to create a negative pressure in the sewage suction channel (L); **characterized in that** the dehumidifying tray (2) comprises:
a tray body, which has a receiving groove formed by a recessed portion, the receiving groove being adapted to the cleaning head for placing a cleaning apparatus in a non-working state; and
a dehumidifying device, which is disposed on the tray body and drives air into the rolling brush chamber to dry the rolling brush when the cleaning apparatus is placed on the tray body;
wherein the dehumidifying device comprises a dehumidifying duct (N1) and a dehumidifying fan (2C), the dehumidifying duct (N1) being in fluid communication with the rolling brush chamber when the cleaning apparatus is placed on the tray body; the dehumidifying fan (2C) is capable of driving the air into the rolling brush chamber through the dehumidifying duct (N1).

2. The dehumidifying tray according to claim 1, **characterized in that** the tray body comprises a main housing (2A) and a duct member (2B), the duct member (2B) being secured to the bottom wall of the main housing (2A) and forming a pre-set gap between the duct member and the bottom wall as a dehumidifying duct (N1), the air outlet of the dehumidifying fan (2C) being fluidly communicated with the dehumidifying duct (N1).

3. The dehumidifying tray according to claim 2, **characterized in that** the receiving groove is located on the upper side of the bottom wall, and the duct member (2B) is secured to the lower side of the main housing (2B), and at least one through-hole penetrating through the bottom wall is formed in the bottom wall, through which the receiving groove is connected to the dehumidifying duct.

4. The dehumidifying tray according to claim 3, **characterized in that** the dehumidifying tray comprises a plurality of the through-holes and the plurality of through-holes being adapted to direct the flow of air to the rolling brush (8H) and the plurality of through-holes being constituted as an air outlet (S1) of the dehumidifying duct (N1).

5. The dehumidifying tray according to claim 3 or claim 4, **characterized in that** the receiving groove comprises a water collection tank (M1) that is further recessed from the bottom thereof, the position of the water collection tank (M1) being adapted to the position of the rolling brush (8H), the through-holes being configured in the wall of the water collection tank (M1), or the through-holes being configured at the transition between the wall of the water collection tank (M1) and the bottom of the receiving groove.

6. The dehumidifying tray according to claim 5, **characterized in that** a sealing member (2F) is disposed between the water collection tank (M1) and the rolling brush chamber to prevent the leakage of air which is configured to dry the rolling brush.

7. The dehumidifying tray according to claim 2 or claim 3, **characterized in that** the main housing (2B) is integrally formed with a duct baffle (2A1) for defining the extent of the area of the dehumidifying duct (N1), and the duct member (2B) is secured to the duct baffle (2A1).

8. The dehumidifying tray according to claim 7, **characterized in that** the dehumidifying fan (2C) is located within the area enclosed by the duct baffle (2A1).

9. The dehumidifying tray according to claim 3 or claim 4, **characterized in that** the dehumidifying duct (N1) has a progressively enlarged front portion to allow air to flow evenly over the length of the rolling brush to the rolling brush (8H), the front portion extending forwardly away from the dehumidifying fan (2C).

10. The dehumidifying tray according to claim 1, **characterized in that** the dehumidifying duct is equipped with a heating device (2G) which heats the air to improve the drying efficiency of the rolling brush.

11. The dehumidifying tray according to claim 1, **characterized in that** the tray body is configured with an anion generator (2H) for sterilization and deodorization, the anion output port of the anion generator is connected to the dehumidifying duct.

12. A cleaning apparatus, wherein the cleaning apparatus comprises a cleaning head (8), a suction device (6) and a sewage suction channel (L), the cleaning head (8) having a rolling brush chamber and a rolling brush (8H) built into the chamber, the rolling brush chamber being located at an upstream position of the sewage suction channel (L), the suction device (6) being located at a downstream position of the sewage suction channel (L) and being configured to generate negative pressure in the sewage suction channel (L), **characterized in that** the cleaning apparatus is adapted to be placed on the dehumidifying tray according to any one of claims 1-11.

13. The cleaning apparatus according to claim 12, **characterized in that** a filter (7) is provided in the sewage suction channel (L) at an upstream position of the suction device, the dehumidifying duct (N1) being in fluid communication with the filter (7); wherein when the cleaning apparatus is placed on the tray body, the air flows from the rolling brush chamber through the sewage suction channel to the filter (7) to dry the filter (7).

14. The cleaning apparatus according to claim 13, **characterized in that** the filter (7) is a HEPA assembly.

15. The cleaning apparatus according to claim 12, **characterized in that** the cleaning head further comprises a humidity sensor (8P); only when the cleaning apparatus is placed on the tray body, the humidity sensor (8P) is capable of detecting the humidity of the air that has flowed through the rolling brush (8H), for generating a control signal for controlling the turning-on or turning-off of the dehumidifying fan.

16. The cleaning apparatus according to claim 15, **characterized in that** the dehumidifying fan (2C) is controlled to turn off when the humidity of the air detected by the humidity sensor (8P) is less than a preset value.

17. The cleaning apparatus according to claim 15 or claim 16, **characterized in that** the humidity sensor (8P) is configured to be located in the sewage suction channel (L) at a downstream position of the rolling brush (8H).

18. The cleaning apparatus according to any one of claims 12-16, **characterized in that** the rolling brush (8H) is configured to rotate in a controlled manner when the cleaning apparatus is placed on the dehumidifying tray so that different areas thereof are opposite to the air outlet of the dehumidifying duct (N1).

19. The cleaning apparatus according to claim 12, **characterized in that** the suction device comprises a fan housing, and the fan housing has a moisture discharge duct (Q1) and a cooling duct (Q2); an air outlet section (Q12) of the moisture discharge duct (Q1) comprises a first air section (Q121) which allows air flows directly to the moisture discharge air outlet (6E2), and a second air section (Q122) that guides air derived from the first air section (Q121) back to the first air section (Q121); the second air section (Q122) is in an arc shape.

20. The fan according to claim 19, **characterized in that** the fan housing comprises an inner housing assembly (6A) and an outer housing assembly (6B), and the outer housing assembly (6B) has a structure for accommodating the inner housing assembly (6A); the air inlet section (Q11) of the moisture discharge duct (Q1) is located in the inner housing assembly (6A), and the air outlet section (Q12) of the moisture discharge duct (Q1) is located in the outer housing assembly ( 6B), the transitional air section (Q13) between the inner housing assembly (6A) and the outer housing assembly (6B) and connecting the air inlet section (Q11) and the air outlet section (Q12) of the moisture discharge duct (Q1).

21. The fan according to claim 12, **characterized in that** the fan housing comprises an inner housing assembly (6A) and an outer housing assembly (6B), and the outer housing assembly (6B) has a structure for accommodating an inner cavity of the inner housing assembly (6A); the air inlet section (Q21) of the cooling duct (Q2) is located in the outer housing assembly (6B), and the air outlet section (Q22) of the cooling duct (Q2) is located in the inner housing assembly (6A) .

22. The fan according to any one of claims 19 to 21, **characterized in that** the air outlet section (Q22) of the cooling duct (Q2) is connected to the air inlet section (Q11) of the moisture discharge duct (Q1).

23. The fan according to claim 20 or 21, **characterized in that** the inner housing assembly (6A) comprises a first inner housing (6C) and a second inner housing (6D); the first inner housing (6C)) has a first cavity (P1) for installing a dehumidification fan and a second cavity (P2) for installing a cooling fan; the first cavity (P1) is located below the second cavity (P2); the second inner housing (6D) is fitted over the lower side of the first inner housing (6C), and the end section of the air outlet section (Q22) of the cooling duct (Q2) is located between the outer side wall of first inner housing (6C) and the inner side wall of the second inner housing (6D).

24. The fan according to claim 20 or 21, **characterized in that** the outer housing assembly (6B) comprises a first housing (6E), a second housing (6F) and a housing cover (6G), the first housing (6E) is installed on an upper side of the second housing (6F) and forms a cavity for accommodating the inner housing assembly (6A); the housing cover (6G) is installed on an upper side of the first housing (6E), and forms the air outlet section (Q12) of the moisture discharge duct (Q1).

25. The fan according to claim 24, **characterized in that** a sealing gasket (6H) is provided between the housing cover (6G) and the first housing (6E).
